# EUROPEAN PATENT SPECIFICATION

(11) **EP 1 668 373 B1**
(45) Date of publication and mention of the grant of the patent: **04.11.2009**
(21) Application number: 04761326.0
(22) Date of filing: 22.09.2004
(51) Int. Cl.: G01N 33/86, C12Q 1/34, C12Q 1/56, C12P 1/00

(54) **METHOD FOR DETECTING PROCOAGULANT PHOSPHOLIPID**
VERFAHREN ZUM NACHWEIS EINES PROKOAGULIERENDEN PHOSPHOLIPIDS
PROCEDE POUR DETECTER DU PHOSPHOLIPIDE PROCOAGULANT

(30) Priority: 22.09.2003 AU 2003905172
(43) Date of publication of application: 14.06.2006
(73) Proprietor: Haematex Research Pty Limited, Hornsby, NSW 2077 (AU)
(72) Inventor: EXNER, Thomas, Gordon, NSW 2072 (AU)
(74) Representative: Desaix, Anne
(86) International application number: PCT/AU2004/001291
(87) International publication number: WO 2005/029093

(56) References cited:
- WO-A-00/23612
- WAGENVOORD, ET AL.: "A Chromatogenic Test to Determine the Procoagulant Phospholipids in Platelet.rich Plasma and Whole Blood" THROMBOSIS RESEARCH. JUN 1974, vol. 4, no. 0, June 1974 (1974-06), pages suppl 1:89-90, XP009073975 ISSN: 0049-3848
- STOCKER, K.: "Anwendung von Schlangengiftproteinen in der Medizin" SCHWEIZERISCHE MEDIZINISCHE WOCHENSCHRIFT. 13 FEB 1999, vol. 129, no. 6, 13 February 1999 (1999-02-13), pages 205-216, XP008053794 ISSN: 0036-7672
- BOFFA MARIE-CLAIRE: "EVALUATION OF THE PHOSPHOLIPID-RELATED PROCOAGULANT ACTIVITY IN PLASMA. A NEW CLUE FOR DETECTING TENDENCY OF THROMBOSIS?" THROMBOSIS RESEARCH, TARRYTOWN, NY, US, vol. 17, no. 3-4, 15 February 1980 (1980-02-15), pages 567-572, XP009073970 ISSN: 0049-3848
- SUNDELL, ET AL.: "In vitro procoagulant and anticoagulant properties of NAJA NAJA NAJA venom" TOXICON : OFFICIAL JOURNAL OF THE INTERNATIONAL SOCIETY ON TOXINOLOGY. SEP 2003, [Online] vol. 42, no. 3, September 2003 (2003-09), pages 239-247, XP009073863 ISSN: 0041-0101 -& [Online] 30 July 2003 (2003-07-30), XP009073863 ISSN: 0041-0101 Retrieved from the Internet: URL:http://www.sciencedirect.com/science?_ ob=ArticleURL&_udi=B6TCS-4961RP8-1&_coverD ate=09%2F30%2F2003&_alid=469021672&_rdoc=1 &_fmt=&_orig=search&_qd=1&_cdi=5178&_sort= d&view=c&_acct=C000049880&_version=1&_urlV ersion=0&_userid=987766&md5=8e7c5b55028e22 bbdebadd8f4e83193a> [retrieved on 2006-10-16]
- WAGENVOORD R.J. ET AL. THROMBOSIS AND HAEMOSTASIS vol. 72, no. 4, 1994, pages 582 - 587, XP003001416
- GEMMELL C.H. ET AL.: 'ASSESSMENT OF MATERIAL-INDUCED PROCOAGULANT ACTIVITY BY A MODIFIED RUSSELL VIPER VENOM COAGULATION TIME TEST' JOURNAL OF BIOMEDICAL MATERIALS RESEARCH vol. 42, no. 4, 15 December 1998, pages 611 - 616, XP001206747
- EXNER T. ET AL. BLOOD COAGULATION AND FIBRINOLYSIS vol. 14, December 2003, pages 773 - 779, XP003001438
- BOFFA M.-C.: 'Assessment of Procoagulant Activity of Phospholipids with a Venom Specific Inhibitor' THROMBOSIS RESEARCH vol. 4, no. SUPPL.1, 1974, USA, pages 89 - 90

## Description

### Technical Field

This invention relates to blood coagulation tests and more particularly relates to an improved method for a marker of thrombosis and platelet activation and a potential thrombotic risk factor.

### Background Art

Procoagulant phospholipids, including, for example, anionic phospholipids such as phosphatidyl serine, have an important role in the blood coagulation mechanism. Procoagulant phospholipids are required in the intrinsic coagulation pathway for conversion of factor X to Xa by factors VIIIa and IXa and also in the common pathway for cleavage of prothrombin to thrombin by factor Xa. They form part of the tissue factor activator complex. In antithrombotic mechanisms they are involved in the activation of protein C by the thrombin / thrombomodulin complex and in the destruction of factor Va by activated protein C.

Low levels of procoagulant phospholipids are typically present in the blood of healthy individuals, probably as microparticles derived from a variety of cells, principally platelets, but these levels increase when platelets become activated, for example, in response to injury and activation of the blood clotting, complement or immunologic mechanisms. In vitro platelets express maximal procoagulant activity after freeze thawing or activation by collagen/thrombin or membrane disrupting agents such as ionophores. Abnormal activation of platelets in vivo occurs during thrombotic episodes, embolism, sepsis, disseminated intravascular coagulation and infarction. Conversely inadequate activation of platelets occurs in certain bleeding disorders such as von Willebrands disease and with various platelet abnormalities.

Procoagulant phospholipids may be traditionally detected in a sample of patient's blood plasma by a coagulation assay, for example, the Russell's Viper Venom Test (hereinafter "RVVT"), although such assays are more conventionally used for diagnosing lupus anticoagulant. The venom used in the RVVT contains metalloproteases that specifically activate factors V and X. After the addition of venom and calcium ions, coagulation proceeds with a near absolute dependence on procoagulant phospholipid in the patient's sample. The amount of procoagulant phospholipid in the patient's sample is determined according to the time required for the test mixture to form fibrin and coagulate and thereby cease to flow in a tube or increase in optical turbidity or block a hole or aperture. The clotting time or time required for a fibrin clot to form may be replaced as an endpoint indicator in this and subsequent descriptions by a chromogenic substrate which yields a readily-detectable coloured product when acted on by the main clotting enzyme, thrombin.

Where a patient is suspected of having a factor deficiency such as insufficient Factor X, V, II or fibrinogen, or is receiving anticoagulant, the patient's sample is typically mixed with a sample of normal human platelet free plasma for the purpose of supplying those factors which are deficient in the sample. This normal human platelet free plasma is typically known as 'substrate plasma'. The substrate plasma used in these assays is ideally platelet free otherwise coagulation will not be absolutely dependent on procoagulant phospholipid contained in the patient's sample.

In RVVT and other coagulation assays, substrate plasma is usually prepared by high speed centrifugation and/or filtration. A principal disadvantage of this procedure is that it is difficult to control the depletion of procoagulant phospholipid from the substrate plasma. Fresh plasma is essential and this is often inconvenient to obtain. Once plasma has been frozen, platelets contained therein are activated and release procoagulant phospholipid. Accordingly, the sensitivity provided by RVVT and other coagulation assays for detection of procoagulant phospholipid in the patient's sample, and the capacity to regulate the specificity of these assays is limited. A further disadvantage is that these processes do not remove some platelet microparticles which may have neutral buoyancy or may be too small to be filtered out.

Another disadvantage of current methods for procoagulant phospholipid determination is their sensitivity to coagulation inhibitors, such as antibodies. These antibodies occur frequently in autoimmune disease, eg. "antiphospholipid syndrome", and cause prolongation of most clotting tests which employ phospholipid-containing reagents and thus give false negative results in current tests for procoagulant phospholipids.

Boffa M.C., 1980 (Thrombosis research 17, pages 567-572, 1980) discloses a method for evaluating the phospholipids-related procoagulant activity in a sample of plasma from a patient. Said method simply consists in adding successively, to high spun plasma (i.e., to a phospholipid depleted plasma prepared by centrifugation), a sample of patient plasma to be analyzed and berus phospholipase. After addition of calcium chloride, clotting time is recorded and compared to that of controls.

Boffa M.C., 1974 (thrombosis research 4, Supplement Number 1, pages 89-90, 1974) discloses a method for determining the procoagulant activity of phospholipids in a sample of blood or plasma from a patient, using a phospholipase A2 as a venom specific phospholipids inhibitor. Said method consists of the following steps: (i) a "normal" plasma is treated with a phospholipase A2 isolated from Vipera berus venom; (ii) the plasma or blood sample to be analyzed is added to the treated plasma; (iii) the recalcification time of the mixture is recorded and compared to that of controls.

### Summary of the Invention

In view of the role of procoagulant phospholipids in the pathogenesis of thrombotic episodes and their potential as markers of platelet activation, there is a need for an improved method for detecting the presence of and the amount of procoagulant phospholipid in a sample.

Therefore, the present application describes a method of determining the amount of procoagulant phospholipid in a sample, method comprising steps (i) to (iii) performed in the following order: (i) forming an admixture of the sample and a substrate plasma which has been rendered free or substantially free of procoagulant phospholipid sufficient to at least reduce the capacity of the substrate plasma to coagulate, wherein said substrate plasma has been rendered free or substantially free of procoagulant phospholipid by treatment with a phospholipase; (ii) contacting the admixture with a reagent for activating coagulation of plasma in conditions where procoagulant phospholipid is the rate limiting component of the mixture; and (iii) determining the clotting time of the admixture.

According to a first aspect of this invention there is provided a method for determining the amount of procoagulant phospholipid in a human sample, the method comprising steps (i) to (iii) performed in the following order: (i) forming an admixture of the human sample and a non-human substrate plasma which has been rendered free or substantially free of procoagulant phospholipid sufficient to at least reduce the capacity of the substrate plasma to coagulate, wherein said substrate plasma has been rendered free or substantially free of procoagulant phospholipid by treatment with a phospholipase; (ii) contacting the admixture with factor Xa in conditions where procoagulant phospholipid is the rate-limiting component of the mixture; and (iii) determining the clotting time of the admixture.

The application also describes a method of determining the amount of activated platelets and platelet microparticles in a sample, the method comprising steps (i) to (iii) performed in the following order: (i) forming an admixture of the sample and a substrate plasma which has been rendered free or substantially free of procoagulant phospholipid sufficient to at least reduce the capacity of the substrate plasma to coagulate; (ii) contacting the admixture with a reagent for activating coagulation of plasma in conditions for permitting procoagulant phospholipid to coagulate the admixture; and (iii) determining the clotting time of the admixture.

According to a second aspect of this invention there is provided a method for determining the amount of activated platelets and platelet microparticles in a human sample, the method comprising steps (i) to (iv) performed in the following order: (i) forming an admixture of the human sample and a non-human substrate plasma which has been rendered free or substantially free of procoagulant phospholipid sufficient to at least reduce the capacity of the substrate plasma to coagulate, wherein said substrate plasma has been rendered free or substantially free of procoagulant phospholipid by treatment with a phospholipase; (ii) contacting the admixture with factor Xa in conditions for permitting procoagulant phospholipid to coagulate the admixture; (iii) determining the clotting time of the admixture; and (iv) quantitating the amount of activated platelets and platelets microparticles in the sample.

The application also describes a method of assessing whether a patient has had a recent thrombotic episode, the method comprising steps (i) to (iii) performed in the following order: (i) forming an admixture of the sample and a substrate plasma which has been rendered free or substantially free of procoagulant phospholipid sufficient to at least reduce the capacity of the substrate plasma to coagulate; (ii) contacting the admixture with a reagent for activating coagulation of plasma in conditions for permitting procoagulant phospholipid to coagulate the admixture; and (iii) determining the clotting time of the admixture.

A thrombotic episode for example may be deep vein thrombosis, embolism or infarction. By "recent" is meant within the time limit that procoagulant phospholipid derived from the thrombotic event may be detected in the circulation. An estimate would be up to 12 hours from such an event if no further platelet activation occurs.

The application also describes a method of producing a substrate plasma for use in determining the level of procoagulant phospholipid in a sample, said method comprising treating substrate plasma with an enzyme for degrading procoagulant phospholipid sufficient to at least reduce the capacity of the substrate plasma to coagulate.

The application also describes a substrate plasma produced by the above-mentioned method of producing a substrate plasma. This includes the concept of incubating a test plasma containing an unknown amount of procoagulant phospholipid alone with phospholipase and comparing the result of a phospholipid-sensitive test before and after such an incubation. A significant prolongation of the test confirms that procoagulant phospholipid had been present without any need for addition of phospholipid free substrate plasma.

The application also describes a kit for determining the level of procoagulant phospholipid in a sample, said kit comprising: (i) a substrate plasma which has been treated with a phospholipase for degrading phospholipid sufficient to at least reduce the capacity of the substrate plasma to coagulate; (ii) a reagent for activating coagulation of plasma in a phospholipid-dependent manner; and (iii) reference preparations containing known levels of procoagulant phospholipid.

According to another aspect of this invention there is provided a kit for determining the level of procoagulant phospholipid in a human sample, said kit comprising: (i) a non-human substrate plasma which has been treated with a phospholipase for degrading phospholipid sufficient to at least reduce the capacity of the substrate plasma to coagulate; (ii) factor Xa; and (iii) reference preparations containing known levels of procoagulant phospholipid.

The reference preparations containing known levels of procoagulant phospholipid may be used as calibrating agents to construct a reference graph.

### Disclosure of the Invention

The application seeks to address the disadvantages identified above and in one embodiment describes a method for determining whether a sample contains detectable procoagulant phospholipid above the lower sensitivity limit of the method and in a second embodiment, how much. The method comprises forming an admixture of the human sample and a non-human substrate plasma which has been rendered free or substantially free of procoagulant phospholipid sufficient to at least reduce the capacity of the substrate plasma to coagulate in a phospholipid-dependent clotting test. The substrate plasma may be rendered free or substantially free of procoagulant phospholipid by treatment with a phospholipase.

The phospholipid-dependent clotting test may be one that is initiated by *Russells viper* venom or the factor X activator from that venom or the phospholipid dependent prothrombin activator from *Pseudonaja Textilis* venom or more preferably factor Xa of human, animal or recombinant origin.

The plasma may be non-human plasma and is preferably non-human plasma and more preferably animal plasma.

The plasma may be rendered free or substantially free of procoagulant phospholipid by for example, treating with an enzyme which degrades phospholipid in the plasma.

For example to prolong the factor Xa activated clotting time of horse plasma from 50 to 120 sec requires 1 hour incubation at 37 °C with 2 x 10⁻⁵ % *Naja nigricollis* venom. Details of various plasma pre-treatment protocols are shown in Example 1 below. The admixture is then contacted with a reagent for activating coagulation of plasma in conditions where the concentration of procoagulant phospholipid influences the clotting time. A determination as to whether the sample comprises procoagulant phospholipid is made by determining when coagulation of the admixture has occurred.

As described herein, the inventor has found that the sensitivity of a clotting test for detecting procoagulant phospholipid in a sample, such as preferably with a factor Xa-based test, is improved by using as a substrate plasma, a composition in which procoagulant phospholipid has been degraded by treatment with phospholipase. More specifically, an admixture comprising a substrate plasma treated with phospholipase was observed to have an increased clotting time, relative to the clotting time of an admixture comprising untreated platelet poor plasma or normally-treated centrifuged plasma. (Example 2). As the same amounts of test plasma and therefore, the same amounts of added procoagulant phospholipid were provided in all admixtures, it follows that the decreased clotting time in the admixture comprising non-treated and centrifuged substrate plasmas was caused by detection of procoagulant phospholipid from both the substrate plasma and the sample. The admixture comprising the treated substrate plasma, in having an increased clotting time, has improved sensitivity because the only procoagulant phospholipid contributed to the admixture and therefore, which is detected in the assay, is derived from the sample.

The results are surprising because many enzymes typically are not capable of activity when added to plasma. This is because plasma is a complex mixture of heterogenous molecules which can prevent enzyme activity. For example, plasma contains proteins which strongly bind to phospholipids such as apolipoproteins, annexins and beta-2-glycoprotein 1 and these may interfere with the availability of substrate for a phospholipase. Further, phospholipases usually require calcium for their enzymatic activity and this is greatly reduced by the citrate anticoagulant normally used in plasma collected for blood clotting tests. Further, plasma also comprises inhibitor molecules capable of inhibiting the activity of specific enzymes. For example, antitrypsin which binds to and inhibits trypsin, antithrombin which inhibits thrombin and antiplasmins which inhibit plasmin activity. Probably the main inhibitor of most phospholipases in human plasma is annexin V.

Typically the substrate plasma is one which has been treated with a phospholipase. An example of such a phospholipase is a basic phospholipase A2. The phospholipase may be produced synthetically, for example by recombinant DNA technology, or may be derived from an organism. For example, the phospholipase may be derived from snake venom. As exemplified herein, phospholipases derived from the venom of *Naja mossambica* and *N nigricollis* are particularly useful for treating the substrate plasma. Other types of venom which are useful are derived from *Agkistrodon halys, Vipera* species, especially *Berus and Russelli, Crotalus durissus, Enhydrina schistosa, Oxyuranus scutellatus* and *Apis melifera.* The main characteristic of venom phospholipases which makes them effective in plasmas is probably their basic character as shown by a high isoelectric pH. Most of the effective venom-derived phospholipases share structural homology.

Other organisms which may provide a phospholipase for use in treating the substrate plasma include *Streptomyces violaceoruber, Vibrio* species, *Clostridium perfringens, Bacillus cereus.*

It follows that as anionic phospholipids, such as phosphatidyl serine are important in thrombosis, typically the enzyme for degrading the procoagulant phospholipid in the substrate plasma should be one capable of degrading phosphatidyl serine in plasma. As noted above, the use of a substrate plasma treated accordingly improves the specificity for detection of phosphatidyl serine in a coagulation assay for detection of procoagulant phospholipid, such as RVVT or factor Xa-based test.

It is to be understood that the substrate plasma for use in the method of the invention does not need to be treated to degrade all phospholipid in it. However the substrate plasma is typically treated so that its capacity to coagulate, when activated by a procoagulant phospholipid-dependent activator of coagulation, for example *Russell 's Viper* Venom, is at least reduced by the degradation of procoagulant phospholipid in the substrate plasma by the enzyme. Typically, the capacity of the substrate plasma to coagulate when activated by such a reagent is reduced when substantially all of the procoagulant phospholipid, mainly phosphatidyl serine component of the phospholipid in the substrate plasma, has been degraded by the enzyme. The treatment of the substrate plasma with 1 x 10⁻⁵ % of a whole *N nigricollis* venom (containing substantially less of the purified enzyme) for about 1 hour at about 37°C is typically sufficient for degrading substantially all of the procoagulant phospholipid in platelet poor substrate plasma by the enzyme. The actual conditions for depleting individual plasmas depends strongly on their initial content of free procoagulant phospholipid and this depends in turn on the degree of contamination by platelets or other cellular debris (eg see Example 1). Thus plasmas containing high levels of platelets require a longer incubation time or a higher concentration of phospholipase than those which are already low in phospholipid. It is preferable to begin with plasmas which are already low in phospholipid. This phospholipase treatment degrades only about 0.001% of the total 0.1% phospholipid in most platelet poor plasmas. Typically, the proportion of free phosphatidyl serine: total phospholipid is about 1:100,000. A phospholipid-sensitive test such as the factor Xa-activated clotting time (hereinafter "XACT") routinely detects 100-1000ng/mL in patient plasmas. It will be understood that a shorter incubation time could be used with a higher concentration of phospholipase and a longer incubation time would be needed with a lower concentration of phospholipase. Thus, 400ng/mL *of N nigricollis* venom (NNV) in normal porcine plasma requires 40 minutes at 37 °C to prolong a factor X activated clotting time from 48 sec to 100 sec whereas 200 ng/mL NNV requires 90 minutes to achieve a similar 100 sec optimal XACT result (See example 1 for more details). It will also be understood that the method of the invention will be most sensitive for procoagulant phospholipid when all procoagulant phospholipid in the substrate plasma has been degraded by treatment with the enzyme.

Because the activity of venoms useful in this invention is progressive in nature it is desirable to stop their interaction with plasma once the phospholipid level has been depleted adequately. This may be done with dilute antisera and antibodies directed against the venom being used. Commercially available antivenoms against the particular class of venom, eg cobra, being used are effective at concentrations from 0.01 to 1 %.

The substrate plasma can be any composition which corrects for a factor or factors that the patient's sample is deficient in. For example, where the patient's sample is deficient in Factor V, the substrate plasma would contain excess Factor V so as to be capable of effecting coagulation of the patient's sample of plasma. Another example of a substrate plasma is one which contains all factors selected from the group consisting of: factor XII, prekallikrein, high molecular weight kininogen, factor XI, factor VIII, factor IX, factor X, factor V, prothrombin, and fibrinogen at functional levels sufficient to compensate for any defects in the admixed sample. Such a substrate plasma would be used in a kaolin or surface-activated clotting time test. When a test employing tissue factor is used as an activator the substrate plasma must contain factors VII, X, V, II and I (fibrinogen) for the same purpose.

When a test such as the *Russell's Viper* Venom Test is to be used, this requires only coagulation factors X and below in the coagulation cascade to be present, ie factors X, factor V, factor II and fibrinogen. Factors above factor X need not be present for a normal result. If a factor Xa-based test is to be used, even factor X is not necessary in the system for a normal result, only factors V, II and I (fibrinogen) are then required. The phospholipid-dependent prothrombin activators from elapidae venoms require no factors above factor **II** (prothrombin) to induce clotting. Thus, if a Taipan venom-based test were to be used only prothrombin and fibrinogen need be provided for a normal result. Fibrinogen or factor I is necessary only to provide a marker for a clotting endpoint. The maximum rate of thrombin generation can be alternatively detected using chromogenic tripeptide substrates which are converted by thrombin to coloured end-products which can be detected spectrophotometrically.

Typically the substrate plasma is derived from citrated blood. Suitable plasmas are those which are known to be effective in promoting coagulation of a human plasma sample, because they provide a factor variably present in the test sample. Examples of such plasmas include most mammalian plasmas. Those which are exemplified herein to be useful in the method of the invention include plasma derived from pig, horse, cow, sheep, goat, camel, monkey, dog, cat, fox, elephant, Ilama, rabbit, mink, racoon, kangaroo, and mixtures thereof.

The difference between the first and second results is proportional to how much procoagulant phospholipid was destroyed by the phospholipase treatment.

However, as antibodies are generated in some humans which have serological activity against human proteins which bind to procoagulant phospholipids, such as beta 2 glycoprotein 1 and prothrombin, (for example lupus inhibitor antibodies), the use of human plasma as a substrate plasma in the method described herein carries with it some unwanted sensitivity to such inhibitors. Consequently such specimens should be assayed for the presence of these antibodies. Where animal plasma is used to provide the substrate plasma, an advantage of the invention is that the method is much less sensitive to antibodies directed against human clotting factors or lupus cofactors than a method based on human plasma. Such antibodies can occur unexpectedly among patients causing confusion and unreliability from existing clotting test methods.

It will be understood that when a test specimen has altered coagulability, particularly where the individual to be tested has been administered with an anti-coagulant, it may be necessary for the substrate plasma to further comprise at least one agent for controlling the capacity of the anti-coagulant to inhibit coagulation. Those agents which are most likely to be useful are ones capable of controlling the capacity of heparin to inhibit coagulation, because heparin is widely used as an anti-coagulant. These agents include protamine sulphate and polybrene or protamine sulphate. However, other agents include antibodies against hirudin and its analogues or other anticoagulant antagonists.

The substrate plasma would normally be used in a liquid or reconstituted form. However for use in a "point of care" device it could be present as part of a dry composition reconstituted by the applied specimen of blood or plasma itself.

The reagent for activating coagulation of the admixture in the test must activate coagulation to proceed subsequently in a procoagulant phospholipid-dependent manner. Examples of such reagents are those capable of converting factor X to factor Xa, or capable of converting prothrombin to thrombin. Accordingly, the reagent for use in the method described herein may be *Russell 's* Viper Venom or factor X activator from a related venom of the viperidae family or factor Xa or other phospholipid-dependent prothrombin activator derived from elapid venoms such as the Australian cobra *Pseudonaja* or *Oxyuranus sculellatus* family. Reagents derived from mammals other than human are particularly useful, for example factor Xa of bovine origin (see Example 4). Reagents acting higher up the coagulation mechanism such as contact activators, tissue factor, factor IXa, factor XIa and factor VIIa can be used, but these make the system less specific for phospholipid and more vulnerable to interference by patient plasma variables.

Clotting activators may also be enzymes from recombinant precursors based on novel DNA sequences. Such procoagulants could be rendered insensitive to inhibiting antibodies by deletion of common epitopes recognised by such antibodies. These reagents would normally be used in liquid form but could also be provided in a dried form for application in a "point of care" device, in which case they would be reconstituted by an applied specimen of plasma or blood specimen.

While it is anticipated that the method described herein would be most widely applied in relation to a plasma or blood sample derived from a human patient. The sample to be tested for procoagulant phospholipid can be blood, plasma, serum or any other fluid. If anticoagulated by calcium-binding agents such as citrate or EDTA, the levels of such agents should be similar to those used in other clotting tests.

In the first aspect of the invention mentioned above, there is provided a method of determining the amount of procoagulant phospholipid in a sample.

The measurement is made in comparison with reference plasmas containing known amounts of procoagulant phospholipid and unknown values may be interpolated from an appropriately constructed calibration curve.

As procoagulant phospholipids are typically located on activated platelets and platelet microparticles, it follows that measuring the amount of procoagulant phospholipid in platelet rich plasma according to the first aspect of the invention would enable one to quantitate the amount of activated platelets and platelet microparticles in the sample.

Thus in the second aspect mentioned above, the invention provides a method of determining the amount of activated platelets and cell-derived microparticles in a sample, the method according to the first aspect of the invention.

As noted above, abnormal platelet or cellular activation may result from thrombotic episodes, embolism, tissue trauma, immune processes (including complement activation), sepsis, disseminated intravascular coagulation or infarction. In extreme cases, or when due to immunologic processes it can result in thrombocytopenia. It would be advantageous to be able to determine whether an individual has a clinical condition involving platelet activation, for example, thrombosis, stroke or myocardial infarction. The inventor recognises that a method for deternmining the amount of activated platelets or platelet microparticles, by determining the amount of procoagulant phospholipid would allow diagnosis of those individuals with those conditions.

In another aspect, the invention provides a method of assessing whether a patient has recently had a thrombotic episode such as a deep vein thrombosis, embolism, infarction, the method according to the second aspect of the invention.

The application also describes a method for producing a substrate plasma for use in determining whether an individual comprises procoagulant phospholipid. The method comprises treating substrate plasma with an enzyme for degrading procoagulant phospholipid sufficient to at least reduce the capacity of the substrate plasma to coagulate.

Using an enzyme in the above-mentioned method for producing a substrate plasma, one is able to provide a panel of substrate plasmas comprising defined amounts of procoagulant phospholipid. This allows one to control the sensitivity of the methods of the first and second aspect of the invention, by selecting for use from the panel, a substrate plasma comprising the desired amount of procoagulant phospholipid. This option provides a reasonable or optimised baseline clotting time for a particular instrument. Snake venoms are particularly useful to provide an enzyme for use in the above-mentioned method for producing a substrate plasma because they can be used at very low concentrations and their activity can be controlled subsequently for example, by the use of antisera and antibodies effective against phospholipases. However, it will be understood that agents capable of controlling phospholipase enzymes derived from recombinant DNA technology, or from other organisms or inhibitory compounds could be used. Thus, a further step of this method comprises contacting the substrate plasma with at least one agent for controlling the capacity of the enzyme to degrade procoagulant phospholipid.

Also, in another embodiment, the above-mentioned method for producing a substrate plasma comprises the further step of mixing the substrate plasma with at least one agent such as Polybrene or protamine sulphate for controlling the capacity of a therapeutic anticoagulant such as heparin to inhibit coagulation.

The application also describes a substrate plasma produced by the above-mentioned method for producing a substrate plasma.

The application also describes a kit for determining whether an individual comprises procoagulant phospholipid, the kit comprising: (i) a substrate plasma which has been treated with an enzyme for degrading phospholipid sufficient to at least reduce the capacity of the substrate plasma to coagulate; and (ii) a reagent for activating coagulation of plasma in a phospholipid-dependent manner (iii) reference preparations containing known levels of procoagulant phospholipid, wherein the reference preparations containing known levels of procoagulant phospholipid may be used as calibrating agents to construct a reference graph.

### Brief Description of the Drawings

The invention is further described with reference to the drawings in which:
Fig 1 is a representation of the effect of incubating normal plasmas from various species with or without *N nigricollis* venom as described in Example 1;
Fig 2 is a representation of the effect of pretreatment with *N nigricollis* venom on platelet sensitivity; and
Fig 3 is a representation of the sensitivity of various tests for platelet phospholipid.

### Best Modes and other modes for carrying out the Invention

The present invention will now be described with reference to the following examples.

### Example 1

### Progressive effect of N nigricollis venom on crude animal-plasmas

Aim: To demonstrate the progressive and selective effect of a typical venom phospholipase in reducing the procoagulant phospholipid from platelet-containing plasmas from various species, thereby improving the sensitivity of those substrate plasmas in clotting tests for procoagulant phospholipid.
Method: Blood samples were collected into one tenth its final volume of 3.2% trisodium citrate anticoagulant by clean venipuncture from a human volunteer, by cardiac puncture from a freshly shot horse (equine), by an arterial bleed from a pig at an abattoir and similarly from an ox (bovine). The samples were centrifuged at 3,000 rpm for 20 minutes and the supernatant platelet poor plasmas with quite variable platelet counts (approximately 5 x 10⁹ /L for the human sample, but not measured for the animal plasmas) were frozen at -30°C.

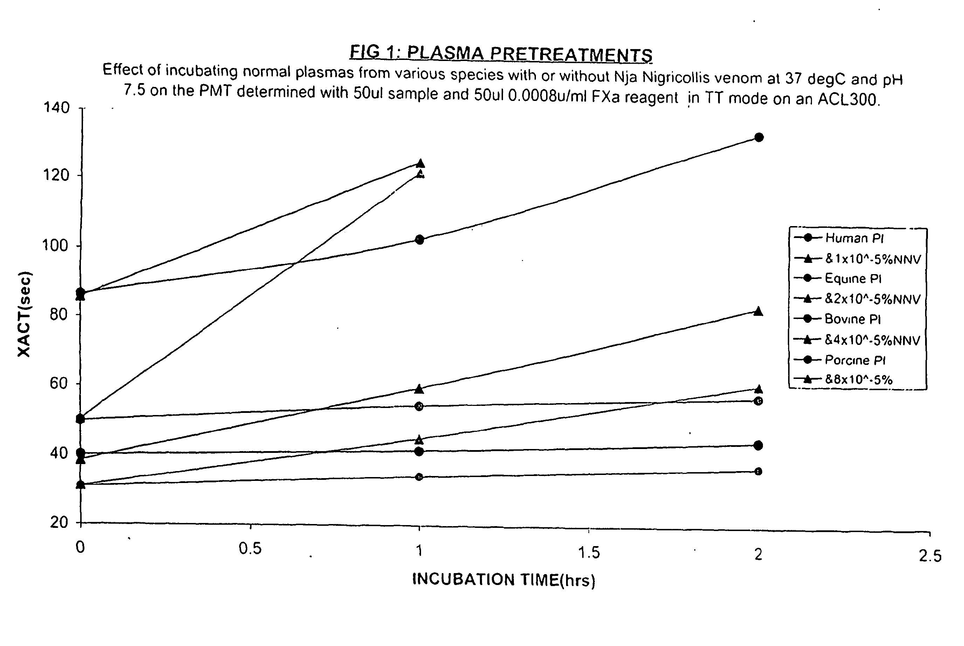

Subsequently thawed platelet poor samples were incubated at 37°C without treatment or after mixing with *N nigricollis* venom (NNV) at the concentrations shown in Fig 1 Specimens were removed at 1 or 2 hour intervals and tested with a factor Xa/calcium reagent for the XACT test
Results: These are shown in Fig 1 XACT results on all plasmas without NNV additions were reasonably stable. With NNV present however XACT results prolonged over the incubation period.
Bovine and porcine plasmas gave the shortest initial results, probably due to excess free procoagulant phospholipid, but these both doubled after incubation for 2 hours with 4 x 10⁻⁵ % and 8 x 10⁻⁵ % NNV. The XACT on horse plasma prolonged from 50 to 120 sec after 1 hour with 2 x 10⁻⁵ % NNV.
Comments: These increases in XACT results due to incubation with NNV were not accompanied by significant changes in activated partial thromboplastin time (APTT), prothrombin time (PT) or other clotting tests. This confirms that the major effect of the NNV was not due to degradation of coagulation factors involved in these clotting tests, but rather to loss of phospholipid for which these tests are not sensitive.

### Example 2

### Pretreatment of human plasma with N nigricollis venom.

Aim: To show that treatment of a normal human plasma with a trace of *N nigricollis* venom gives a product (substrate plasma) with better sensitivity to platelets in a Factor Xa-based clotting test than centrifugation.
Method: Test plasmas containing varying levels of freeze-thawed normal platelet rich plasma (PRP initially with 250 x 10⁹ platelets/L) in platelet "free" normal human plasma were prepared. The platelet free plasma (PFP) was obtained by high speed centrifugation and filtration through a 0.22 micron syringe filter.

These test plasmas were mixed with an equal volume of 3 different substrate plasmas before being tested in a factor Xa-based clotting test. The 3 different substrate plasmas were:
1. Normal platelet "poor" human plasma (PPP).
2. The same PPP centrifuged at 15,000g for 10 min.
3. The same PPP treated with 1 x 10⁻⁵ % *N nigricollis* venom for 20 minutes at 37 °C (hereinafter the "NNV treatment").

The factor Xa reagent contained 0.001 U/mL bovine Factor Xa in 0.015 M calcium chloride, 0.1M sodium chloride, 0.02M HEPES pH 7.0 buffer and was used in a proportion of 0.05 mL plasma mix with 0.1 mL of reagent in a ST4 (Diagnostica Stago, Paris, France) clotting machine at 37°C.
Results: Table 1 shows Factor Xa clotting time results in seconds on 1:1 mixes of test plasmas containing various platelet counts and substrate pooled normal plasma (PNP) pretreated by the two different methods.

**Table 1**

| **Test Plasmas Substrate Plasmas** | | | |
|---|---|---|---|
| Platelet count (10⁹/L) | PNP initially | Centrifuged PNP | PNP after NNV treatment |
| 25 | 48.2 | 45.9 | 50.8 |
| 5 | 58.9 | 66.2 | 73.9 |
| 1 | 64.1 | 85.7 | 96.4 |
| < 0.2 (PFP) | 67.7 | 95.4 | 117 |

Comment: These experiments show that NNV treatment achieved a greater increase in clotting time results over those obtained with high speed centrifugation. This resulted in an improvement in sensitivity to platelets.

### Example 3

### Effect of a pre-treatment with N nigricollis venom on platelet sensitivity.

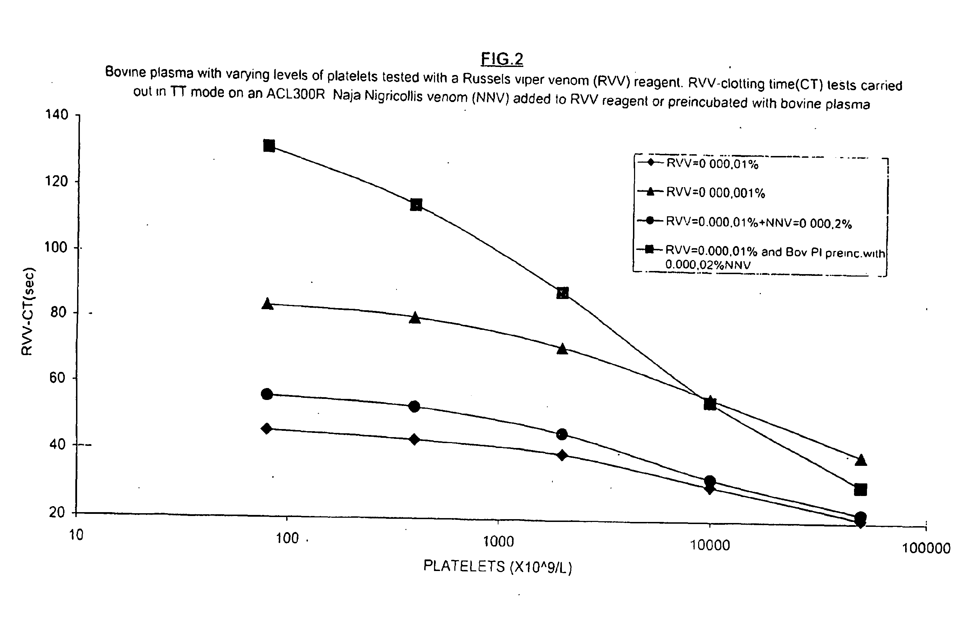

Aim: To demonstrate the effect of *N nigricollis* venom in enhancing the sensitivity of a Russells viper venom clotting test system based on bovine plasma.
Method: A series of dilutions of a frozen-thawed, though otherwise normal human platelet rich plasma (with initial platelet count of 250 x 10⁹/L) were made in normal bovine plasma and also in bovine plasma pretreated for 50 min at 20°C with 5 x 10^{-5%} *N nigricollis* venom. These plasma samples were mixed with an equal volume of various *Russell's viper* venom and calcium-containing reagents and timed to a clotting endpoint at 37°C in thrombin time mode (TT mode uses equal volumes of plasma and reagent) in a ACL300 clot-timing instrument (Instrumentation Laboratory SpA, Milan, Italy). The *Russell's viper* venom concentration in the reagent with 0.025M calcium chloride was varied from 10⁻⁵% to 10⁻⁶% and the former reagent was also tested after the addition of 2 x 10⁻⁴*% N nigricollis* venom.
Results: The results obtained are summarised in Fig 2. It is apparent that the sensitivity of a test system using 1 x 10⁻⁵% RVV to platelets was quite low, plateauing out at platelet levels below 1 x 10⁹/L. RVV clotting times were prolonged by reducing the RW concentration tenfold to 10⁻⁶%, but sensitivity to platelets as shown by the gradient of the responsiveness curve was not improved. Including 20 x 10⁻⁵% NNV in the RVV reagent (RW= 10⁻⁵%) increased the sensitivity slightly.

The highest sensitivity to platelets was observed when the bovine plasma had been preincubated with 5 x 10⁻⁵ % NNV before being used to dilute out the platelet concentrate. In this case platelet counts between 0.1 and 1.0 could still be quantitated accurately.

### Example 4

### Comparison of various clotting activators

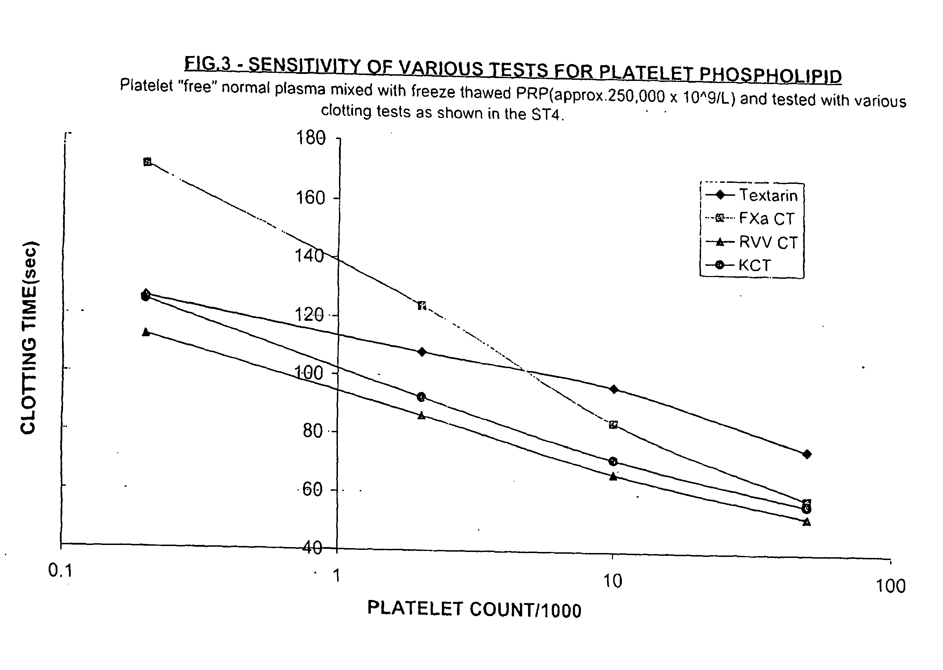

Aim: To compare the sensitivities of 4 different phospholipid-dependent clotting activators in a test system for assaying procoagulant phospholipid.
Method: Dilutions of a platelet rich plasma were prepared in platelet free normal human plasma as shown below. These samples were tested with 4 different clotting test systems. All tests were carried out at 37°C in a ST4. The reagents and methods were as follows.
1. Kaolin clotting tests (KCT) were carried out using 0.05 mL plasma samples preincubated with 0.05 mL of 1% kaolin suspension in water for 3 min and then recalcified with 0.05 mL of 0.025 M calcium chloride. The time from addition of calcium chloride till clotting occurred was determined in a ST4 (Stago) clotting machine.
*2. Russell's Viper* Venom Tests (RVV) were carried out by mixing 0.05 mL samples with 0.05 mL of a reagent containing 2 x 10⁻⁶ % RVV in 0.025M calcium chloride and timing till a clotting endpoint.
3. Factor Xa-based clotting tests (FXa-CT) were carried out by mixing 0.05 mL samples with 0.05 mL of a reagent containing 0.001 U/mL bovine factor Xa in 0.025 M calcium chloride and timing to a clotting endpoint.
4. Textarin (TM-Pentapharm, Basel, Switzerland) clotting tests (TX-CT) were carried out by mixing 0.05 mL samples with 0.05 mL of a reagent containing 2 U/mL of delipidated commercial Textarin reagent in 0.025 M calcium chloride and timing to a clotting endpoint.
   Results: Results obtained are shown in Fig 3. The RVVT and KCT tests showed similar sensitivities to platelets. The clotting test based on activated factor X (XACT) showed the highest sensitivity to platelets. The test with the lowest sensitivity to platelets was that based on delipidated Textarin. However it is possible that this may have been due to inadequate removal of phospholipid from this commercial reagent intended for an alternative purpose, ie. detection of lupus inhibitors.
   Comments: The Textarin reagent is a typical phospholipid-dependent prothrombin activator as derived from the venom of *Pseudonaja textilis,* one of several Australian elapids known to contain such procoagulants.

### Example 5

### Typical use of the method and specificity study

Aim: To illustrate that the method is insensitive to defects in all known clotting factors. Also to detect free procoagulant phospholipid in various commercially-available plasmas deficient in individual clotting factors.
Method: Various freeze-dried individual clotting factor deficient plasmas marketed for use in specific factor assays were tested using the new test for procoagulant phospholipid. Thus the vials from various suppliers (DadeBehring, IL/Beckman-Coulter and Diagnostica Stago) were each freshly reconstituted with 1 mL of water. The tests used 25 µl of NNV-treated substrate plasma (lot 3004) with 25 µl of each factor deficient plasma and 50 µl of factor Xa reagent in a Stago ST4 clotting machine.
Results: These are shown in Table 2.

**Table 2**

| Test Plasma | FXa-Clotting Time(Sec) |
|---|---|
| Frozen"platelet-poor" plasma | 53.7 |
| Platelet "free" normal plasma | 102 |
| Prothrombin(Fli) deficient | 88.8 |
| Factor V deficient plasma | 100 |
| Factor VII deficient | 96.5 |
| Factor VIII deficient | 70.0 |
| Factor IX deficient | 71.6 |
| Factor X deficient | 73.7 |
| Factor XI deficient | 88.3 |
| Factor XII deficient | 96.7 |

Comment: The pooled frozen platelet-poor plasma gave a relatively short FXa clotting time compared with the platelet free normal plasma because it contained approximately 5% of a normal platelet count (approximately 10 x 10⁹ platelets/L).

These results show that the total deficiency of any individual plasma clotting factor in a test sample does not prolong the FXa test. It also shows that the factor VIII, IX and X deficient plasmas used here contain appreciable amounts of procoagulant phospholipid detectable with this test.

### Industrial Applicability

It should be clear that the methods of the present invention will find wide application in clinical laboratory science.

## Claims

1. A method for determining the amount of procoagulant phospholipid in a human sample, said method comprising steps (i) to (iii) performed in the following order:
(i) forming an admixture of the human sample and a non-human substrate plasma which has been rendered free or substantially free of procoagulant phospholipid sufficient to at least reduce the capacity of the substrate plasma to coagulate, wherein said substrate plasma has been rendered free or substantially free of procoagulant phospholipid by treatment with a phospholipase;
(ii) contacting the admixture with factor Xa in conditions where procoagulant phospholipid is the rate limiting component of the mixture; and
(iii) determining the clotting time of the admixture.

2. A method according to claim 1 wherein the sample is selected from the group consisting of blood, plasma and serum.

3. A method according to claim 2 wherein said blood is anticoagulated blood.

4. A method according to claim 1 wherein the measurement is made in comparison with reference plasmas or solutions containing known amounts of procoagulant phospholipid and unknown values may be interpolated from an appropriately constructed calibration curve.

5. The method of claim 1, wherein said substrate plasma is derived from citrated blood.

6. The method of claim 1 wherein said substrate plasma is obtained from a member selected from the vertebrate animal group consisting of pig, horse, cow, sheep, goat, camel, monkey, dog, cat, fox, elephant, llama, rabbit, mink, racoon, kangaroo, and mixtures thereof.

7. The method of claim 6, wherein said substrate plasma is obtained from pigs.

8. The method of claim 1, wherein the phospholipase is obtained from venom selected from the group consisting of Naja *mossambica, Naja nigricollis, Agkistrodon halys, Vipera Berus, Vipera Russeli, Crotalus durissus, Enhyrdrina schistose, Oxyuranus scutellatus* and *Apis mellifera.*

9. The method of claim 1, wherein the phospholipase is obtained from one of a selected group consisting of *Streptomyces violaceoruber, Vibrio* species, *Clostridium perfringens,* or *Bacillus cereus.*

10. The method of claim 1, wherein factor Xa is derived from a mammal other than a human.

11. A method for determining the amount of activated platelets and platelet microparticles in a human sample, said method comprising steps (i) to (iv) performed in the following order:
(i) forming an admixture of the human sample and a non-human substrate plasma which has been rendered free or substantially free of procoagulant phospholipid sufficient to at least reduce the capacity of the substrate plasma to coagulate, wherein said substrate plasma has been rendered free or substantially free of procoagulant phospholipid by treatment with a phospholipase;
(ii) contacting the admixture with factor Xa in conditions for permitting procoagulant phospholipid to coagulate the admixture;
(iii) determining the clotting time of the admixture; and
(iv) quantitating the amount of activated platelets and platelets microparticles in the sample.

12. A method according to claim 11, wherein said method determines if a patient has had a recent thrombotic episode.

13. A method according to claim 11, wherein said method determines if a patient has had a clinical disorder involving platelet activation.

14. The method according to claim 12, wherein the thrombotic episode is selected from the group consisting of disseminated intravascular coagulation, deep vein thrombosis, embolism, tissue trauma, sepsis, and infarction.

15. The method of claim 11, wherein the phospholipase is obtained from venom selected from the group consisting of *Naja mossambica, Naja nigricollis, Agkistrodon halys, Vipera Berus, Vipera Russeli, Crotalus durissus, Enhyrdrina schistose, Oxyuranus scutellatus and Apis mellifera.*

16. The method of claim 11, wherein the phospholipase is obtained from one of a selected group consisting of *Streptomyces violaceoruber, Vibrio* species, *Clostridium perfringens,* or *Bacillus cereus.*

17. The method of claim 11, wherein factor Xa is derived from a mammal other than a human.

18. The method of claim 11, wherein the substrate plasma has been formed from factors V and prothrombin.

19. The method of claim 18, wherein said factors V and prothrombin are phospholipid free.

20. The method of claim 18, wherein said factors V and prothrombin are of animal or human origin.

21. The method of any one of claims 11 to 20, wherein said substrate plasma is obtained from a member selected from the group consisting of pig, horse, cow, sheep, goat, camel, monkey, dog, cat, fox, elephant, llama, rabbit, mink, racoon, kangaroo, and mixtures thereof.

22. A kit for determining the level of procoagulant phospholipid in a human sample, said kit comprising:
(i) a non-human substrate plasma which has been treated with a phospholipase for degrading phospholipid sufficient to at least reduce the capacity of the substrate plasma to coagulate;
(ii) factor Xa; and
(iii) reference preparations containing known levels of procoagulant phospholipid.

## Patentansprüche

1. Verfahren zum Bestimmen der Menge eines Prokoagulationsphospholipids in einer humanen Probe, wobei das Verfahren die Schritte (i) bis (iii) umfasst, die in der folgenden Reihenfolge durchgeführt werden:
(i) Bilden einer Mischung der humanen Probe und eines nicht-humanen Substratplasmas, das frei oder im Wesentlichen frei von Prokoagulationsphospholipid gemacht worden ist, ausreichend, um das Koagulatiosvermögen des Substratplasmas wenigstens zu verringern, wobei das Substratplasma durch eine Behandlung mit einer Phospholipase frei oder im Wesentlichen frei von Prokoagulationsphospholipid gemacht worden ist;
(ii) Kontaktieren der Mischung mit Faktor Xa unter Bedingungen, unter denen das Prokoagulationsphospholipid die geschwindigkeitslimitierende Komponente der Mischung ist; und
(iii) Bestimmen der Gerinnungszeit der Mischung.

2. Verfahren nach Anspruch 1, wobei die Probe aus der Gruppe bestehend aus Blut, Plasma und Serum ausgewählt ist.

3. Verfahren nach Anspruch 2, wobei das Blut antikoaguliertes Blut ist.

4. Verfahren nach Anspruch 1, wobei die Messung im Vergleich mit Referenzplasmen oder -lösungen, die bekannte Mengen an Prokoagulationsphospholipid enthalten, erfolgt und nicht bekannte Werte aus einer geeignet aufgestellten Kalibrationskurve interpoliert werden können.

5. Verfahren nach Anspruch 1, wobei das Substratplasma von Citratblut stammt.

6. Verfahren nach Anspruch 1, wobei das Substratplasma von einem Mitglied ausgewählt aus der Wirbeltiergruppe bestehend aus Schwein, Pferd, Kuh, Schaf, Ziege, Kamel, Affe, Hund, Katze, Fuchs, Elefant, Lama, Kaninchen, Nerz, Waschbär, Känguru und Mischungen davon erhalten wird.

7. Verfahren nach Anspruch 6, wobei das Substratplasma von Schweinen erhalten wird.

8. Verfahren nach Anspruch 1, wobei die Phospholipase aus einem Schlangengift ausgewählt aus der Gruppe bestehend aus *Naja mossambica, Naja nigricollis, Agkistrodon halys, Vipera Berus, Vipera Russeli, Crotalus durissus, Enhyrdrina schistose, Oxyuranus scutellatus* und *Apis mellifera* erhalten wird.

9. Verfahren nach Anspruch 1, wobei die Phospholipase von einem aus einer ausgewählten Gruppe bestehend aus *Streptomyces violaceoruber, Vibrio* Spezies, *Clostridium perfringensoder Bacillus cereuserhalten* wird.

10. Verfahren nach Anspruch 1, wobei Faktor Xa von einem Säugetier, das kein Mensch ist, stammt.

11. Verfahren zum Bestimmen der Menge von aktivierten Blutplättchen und Blutplättchen-Mikropartikeln in einer humanen Probe, wobei das Verfahren die Schritte (i) bis (iv) umfasst, die in der folgenden Reihenfolge durchgeführt werden:
(i) Bilden einer Mischung der humanen Probe und eines nicht-humanen Substratplasmas, das frei oder im Wesentlichen frei von Prokoagulationsphospholipid gemacht worden ist, ausreichend, um das Koagulationsvermögen des Substratplasmas wenigstens zu verringern, wobei das Substratplasma durch eine Behandlung mit einer Phospholipase frei oder im Wesentlichen frei von Prokoagulationsphospholipid gemacht worden ist;
(ii) Kontaktieren der Mischung mit Faktor Xa unter Bedingungen, unter denen das Prokoagulationsphospholipid die geschwindigkeitslimitierende Komponente der Mischung ist; und
(iii) Bestimmen der Gerinnungszeit der Mischung; und
(iv) mengenmäßiges Bestimmen der Menge von aktivierten Blutplättchen und Blutplätchen-Mikropartikeln in der Probe.

12. Verfahren nach Anspruch 11, wobei das Verfahren bestimmt, ob ein Patient kürzlich ein thrombotisches Ereignis gehabt hat.

13. Verfahren nach Anspruch 11, wobei das Verfahren bestimmt, ob ein Patient eine klinische Erkrankung, an der eine Blutplättchenaktivierung beteiligt ist, gehabt hat.

14. Verfahren nach Anspruch 12, wobei das thrombotische Ereignis aus der Gruppe bestehend aus disseminierter intravasaler Gerinnung, tiefer Venenthrombose, Embolie, Gewebstrauma, Sepsis und Infarkt ausgewählt ist.

15. Verfahren nach Anspruch 11, wobei die Phospholipase aus einem Schlangengift ausgewählt aus der Gruppe bestehend aus *Naja mossambica, Naja nigricollis, Agkistrodon halys, Vipera Berus, Vipera Russeli, Crotalus durissus, Enhyrdrina, schistose, Oxyuranus scutellatus* und *Apis mellifera* erhalten wird.

16. Verfahren nach Anspruch 11, wobei die Phospholipase aus einem aus einer ausgewählten Gruppe bestehend aus *Streptomyces violaceoruber, Vibrio* Spezies, *Clostridium perfringens* oder *Bacillus cereus* erhalten wird.

17. Verfahren nach Anspruch 11, wobei Faktor Xa von einem Säugetier, das kein Mensch ist, stammt.

18. Verfahren nach Anspruch 11, wobei das Substratplasma aus Faktoren V und Prothrombin gebildet worden ist.

19. Verfahren nach Anspruch 18, wobei die Faktoren V und Prothrombin phospholipidfrei sind.

20. Verfahren nach Anspruch 18, wobei die Faktoren V und Prothrombin tierischen oder humanen Ursprungs sind.

21. Verfahren nach einem der Ansprüche 11 bis 20, wobei das Substratplasma von einem Mitglied ausgewählt aus der Gruppe bestehend aus Schwein, Pferd, Kuh, Schaf, Ziege, Kamel, Affe, Hund, Katze, Fuchs, Elefant, Lama, Kaninchen, Nerz, Waschbär, Känguru und Mischungen davon erhalten wird.

22. Kit zum Bestimmen des Spiegels von Prokoagulationsphospholipid in einer humanen Probe, wobei das Kit umfasst:
(i) ein nicht-humanes Substratplasma, das mit einer Phospholipase zum Abbauen von Phospholipid behandelt worden ist, ausreichend, um das Koagulationsvermögen des Substratplasmas wenigstens zu verringern;
(ii) Faktor Xa; und
(iii) Referenzpräparate, die bekannte Spiegel von Prokoagulationsphospholipid enthalten.

## Revendications

1. Procédé de détermination de la quantité de phospholipide procoagulant dans un échantillon humain, ledit procédé comprenant les étapes (i) à (iii) exécutées dans l'ordre suivait :
(i) formation d'un mélange de l'échantillon humain et d'un plasma substrat non humain qui a été rendu dépourvu ou substantiellement dépourvu de phospholipide procoagulant suffisamment pour au moins réduire la capacité du plasma substrat à coaguler, dans lequel ledit plasma substrat a été rendu dépourvu ou substantiellement dépourvu de phospholipide procoagulant par traitement avec une phospholipase ;
(ii) mise en contact du mélange avec le facteur Xa dans des conditions dans lesquelles le phospholipide procoagulant est le composant cinétiquement limitant du mélange ; et
(iii) détermination du temps de coagulation du mélange.

2. Procédé selon la revendication 1 dans lequel l'échantillon est choisi parmi le groupe consistant en le sang, le plasma et le sérum.

3. Procédé selon la revendication 2 dans lequel ledit sang est du sang anticoagulé.

4. Procédé selon la revendication 1 dans lequel la mesure est effectuée en comparaison avec des plasmas ou des solutions de référence contenant des quantités connues de phospholipide procoagulant et des valeurs inconnues peuvent être interpolées à partir d'une courbe d'étalonnage construite de façon appropriée.

5. Procédé selon la revendication 1 dans lequel ledit plasma substrat est obtenu à partir de sang citraté.

6. Procédé selon la revendication 1 dans lequel ledit plasma substrat est obtenu à partir d'un élément choisi parmi le groupe des animaux vertébrés consistant en le porc, le cheval, la vache, le mouton, la chèvre, le chameau, le singe, le chien, le chat, le renard, l'éléphant, le lama, le lapin, le vison, le raton laveur, le kangourou, et des mélanges de ceux-ci.

7. Procédé selon la revendication 6, dans lequel ledit plasma substrat est obtenu à partir de porcs.

8. Procédé selon la revendication 1, dans lequel la phospholipase est obtenue à partir de venin choisi parmi le groupe consistant en *Naja mossambica, Naja nigricollis, Agkistrodon halys, Vipera Berus, Vipera Russeli, Crotalus durissus, Enhyrdrina schistose, Oxyuranus scutellatus* et *Apis mellifera.*

9. Procédé selon la revendication 1, dans lequel la phospholipase est obtenue à partir de l'un d'un groupe choisi consistant en *Streptomyces violaceoruber,* l'espèce *Vibri o, Clostridium perfringens,* ou *Bacillus cereus .*

10. Procédé selon la revendication 1, dans lequel le facteur Xa est obtenu à partir d'un mammifère autre qu'un humain.

11. Procédé de détermination de la quantité de plaquettes et de microparticules de plaquettes activées dans un échantillon humain, ledit procédé comprenant les étapes (i) à (iv) exécutées dans l'ordre suivant :
(i) formation d'un mélange de l'échantillon humain et d'un plasma substrat non humain qui a été rendu dépourvu ou substantiellement dépourvu de phospholipide procoagulant suffisamment pour au moins réduire la capacité du plasma substrat à coaguler, dans lequel ledit plasma substrat a été rendu dépourvu ou substantiellement dépourvu de phospholipide procoagulant par traitement avec une phospholipase ;
(ii) mise en contact du mélange avec le facteur Xa dans des conditions permettant que le phospholipide procoagulant coagule le mélange ;
(iii) détermination du temps de coagulation du mélange ; et
(iv) quantification de la quantité de plaquettes et de microparticules de plaquettes activées dans l'échantillon.

12. Procédé selon la revendication 11, dans lequel ledit procédé détermine si un patient a eu un épisode thrombotique récent.

13. Procédé selon la revendication 11, dans lequel ledit procédé détermine si un patient a eu un trouble clinique impliquant l'activation des plaquettes.

14. Procédé selon la revendication 12, dans lequel l'épisode thrombotique est choisi parmi le groupe consistant en une coagulation intravasculaire disséminée, une thrombose veineuse profonde, une embolie, un trauma tissulaire, une sepsie, et un infarctus.

15. Procédé selon la revendication 11, dans lequel la phospholipase est obtenue à partir de venin choisi parmi le groupe consistant en *Naja mossambica, Naja nigricollis, Agkistrodon halys, Vipera Berus, Vipera Russeli, Crotalus durissus, Enhyrdrina schistose, Oxyuranus scutellatus* et *Apis mellifera.*

16. Procédé selon la revendication 11, dans lequel la phospholipase est obtenue à partir de l'un d'un groupe choisi consistant en *Streptomyces violaceoruber,* l'espèce *Vibrio, Clostridium perfringens,* ou *Bacillus cereus.*

17. Procédé selon la revendication 11, dans lequel le facteur Xa est dérivé d'un mammifère autre qu'un humain.

18. Procédé selon la revendication 11, dans lequel le plasma substrat a été formé à partir de facteurs V et de prothrombine.

19. Procédé selon la revendication 18, dans lequel lesdits facteurs V et ladite prothrombine sont dépourvus de phospholipide.

20. Procédé selon la revendication 18, dans lequel lesdits facteurs V et ladite prothrombine sont d'origine animale ou humaine.

21. Procédé selon l'une quelconque des revendications 11 à 20, dans lequel ledit plasma substrat est obtenu à partir d'un élément choisi parmi le groupe consistant en le porc, le cheval, la vache, le mouton, la chèvre, le chameau, le singe, le chien, le chat, le renard, l'éléphant, le lama, le lapin, le vison, le raton laveur, le kangourou, et des mélanges de ceux-ci.

22. Kit de détermination du niveau de phospholipide procoagulant dans un échantillon humain, ledit kit comprenant :
(i) un plasma substrat non humain qui a été traité avec une phospholipase pour dégrader le phospholipide suffisamment pour au moins réduire la capacité du plasma substrat à coaguler ;
(ii) du facteur Xa ; et
(iii) des préparations de référence contenant des niveaux connus de phospholipide procoagulant.
